Europäisches Patentamt

European Patent Office

Office européen des brevets

(11) Publication number: **0 020 753**
**B1**

# EUROPEAN PATENT SPECIFICATION

(45) Date of publication of patent specification: **04.09.85**

(21) Application number: **80900201.7**

(22) Date of filing: **26.12.79**

(86) International application number:
**PCT/US79/01120**

(87) International publication number:
**WO 80/01350 10.07.80 Gazette 80/15**

(51) Int. Cl.⁴: **A 01 N 1/02, A 61 L 15/04,**
**C 12 N 5/00**

(54) **SKIN-EQUIVALENT.**

(30) Priority: **26.12.78 US 972832**

(43) Date of publication of application:
**07.01.81 Bulletin 81/01**

(45) Publication of the grant of the patent:
**04.09.85 Bulletin 85/36**

(84) Designated Contracting States:
**DE FR GB**

(73) Proprietor: **MASSACHUSETTS INSTITUTE OF TECHNOLOGY**
**77 Massachusetts Avenue**
**Cambridge, MA 02139 (US)**

(72) Inventor: **BELL, Eugene**
**1150 High Street**
**Dedham, MA 02026 (US)**

(74) Representative: **Harvey, David Gareth et al**
**Graham Watt & Co. Riverhead**
**Sevenoaks Kent TN13 2BN (GB)**

(56) References cited:

Journal of The National Cancer Institute Vol. 16, No. 6, issued June 1956, R.L. Ehrmann et al., "The Growth Of Cells On A Transparent Gel Of Reconstituted Rat Tail Collagen", See Pages 1375-1403

Experimental Cell Research, Vol, 26, issued 1962, W.D. Hillis et al, "The Cultivation Of Human Embryonic Liver Cells". See Pages 9-36

The Journal of Cell Biology, Vol. 54, issued 1972, T. Elsade et al., "Collagen Substrata For Studies On Cell Behavior", See Pages 626-637

(56) References cited:

L.J. Mullins et al, Annual Review of Biophysics and Bioengineering, Vol. 3, Published 1974 by Annual Reviews Inc. (Palo Alto, California), K.H. Stenzel et al, "Collagen As A Biomaterial" See Pages 231-253

Experimental Cell Research, Vol. 84 issued 1974, G.O. Gey et al., "Long-Term Growth of Chicken Fibro-Blasts on a Collagen Substrate", See Pages 63-71

Experimental Cell Research, Vol. 94 issued 1975, G. Michalopoulos et al., "Primary Culture of Parenchymal Liver Cells On Collagen Membranes", See pages 70-78

## Description

### Technical Field

This invention is in the field of biology and particularly relates to a material which can be used to cover wounds to skin.

### Background Art

In many wounds which involve injury to a significant area of human skin, and particularly in the case of massive burns, there is an immediate need to cover the wound with a material which provides some of the functions of skin. These functions involve the reduction of fluid loss, prevention of infections, and reduction of the area for potential scarring.

Approaches which have been employed in solving this problem involve the use of homografts, modified dermal xenografts, synthetic polymeric structures, or reconstituted collagen films. While each of these approaches offers partial success, each also has been replete with serious problems which have not been solved. Particularly significant problems in many of these approaches have been rejection of the skin substitute, particularly in the absence of immunosuppressive agents, or breakdown of the graft by host enzymes.

### Disclosure of the Invention

This invention relates to the discovery that a living tissue can be formed. This living tissue is produced by forming a hydrated collagen lattice, *in vitro*, and plating fibroblast cells into the lattice.

The method according to the invention is characterised by:

a. forming a hydrated collagen lattice;

b. bringing fibroblast cells into contact with said collagen lattice; and

c. maintaining said lattice and said cells under conditions sufficient for said cells to attach to said collagen lattice and to contract said collagen lattice thereby forming living tissue.

A convenient technique for simultaneously forming the lattice and plating cells therein involves neutralizing an acidic collagen solution contained in a culture dish with nutrient medium and fibroblast cells. Upon raising the pH of the solution sufficiently, collagen fibrils precipitate from the solution to form the lattice with fibroblast cells homogeneously dispersed therethrough. The cells and collagen lattice are then maintained under conditions which allow the cells to attach to the collagen lattice and to contract it, to a fraction of its original size, thereby providing the living tissue.

A skin-equivalent according to this invention can be produced from this living tissue by plating keratinocyte cells upon the living tissue and providing for their growth. This skin-equivalent is uniquely different from previously described artificial skins because its basic organization is like that of skin and its living constituent cells can be donated by a potential graft recipient.

### Brief Description of the Drawings

FIG. 1 is a plot of data illustrating the contraction of a hydrated collagen lattice;

FIG. 2 is a plot of data illustrating the contraction of hydrated collagen lattices having different collagen contents;

FIG. 3 is a plot of data illustrating the contraction of hydrated collagen lattices containing different numbers of fibroblast cells;

FIG. 4 is a plot of data indicating the contractile capacity in hydrated collagen lattices of cells of different population doubling levels;

FIG. 5 is a plot of data illustrating the effect of 10.0 µg/ml of the inhibitor cytochalasin B on the capacity of cells to contract a hydrated collagen lattice;

FIG. 6 is a plot of data illustrating the effect of 0.36 µg/ml of the inhibitor colcemid on the capacity of cells to contract a hydrated collagen lattice; and,

FIG. 7 is a plot of data illustrating the effect of cytosine arabinoside on the capacity of cells to contract a hydrated collagen lattice.

### Best Mode of Carrying Out the Invention

Hydrated collagen lattices have been prepared employing collagen derived from rat tail tendon and calf skin collagen. Other sources of collagen would be suitable, of course. Solutions of collagen were prepared and maintained under slightly acidic conditions. Lattices were formed by adding fibroblast cells with nutrient medium and base which raised the pH sufficiently to precipitate collagen fibrils from solution. Preparation of hydrated collagen lattices is described in more detail in the following references, the teachings of which are incorporated by reference: Elsdale, T. and Bard, J., "Collagen Substrata for Studies on Cell Behavior," *J. Cell Bio. 54*, 626—637 (1972); Ehrmann, R. L. and Gey, G. O., "The Growth of Cells on A Transparent Gel of Reconstituted Rat-Tail Collagen," *J. Natl. Cancer Inst., 16*, 1375—1403 (1956); Emermann, J. T. and Pitelka, D. R., "Hormonal Effects on Intracellular and Secreted Casein in Cultures of Mouse Mammary Epithelial Cells on Floating Collagen Membranes," *In Vitro, 13*, 316—328 (1977); Gey, G. O., Svotelis, M., Foard, M. and Bang, F. B., "Long-Term Growth of Chicken Fibroblasts On a Collagen Substrate," Exp. Cell Res., 84, 63—71 (1974); and Hillis, W. D. and Band, F. B., "The Cultivation of Human Embryonic Liver Cells," *Exp. Cell Res.*, 26, 9—36 (1962).

The preparation of hydrated collagen lattices is described in a paper by Michalopoulous, G. and Pitot, H. C. in *Exp. Cell Res., 94*, pages 70 to 78 (1975). This paper reports in vitro studies of rat liver parenchymal cells. The liver cells were plated — as a monolayer only — onto collagen as a support therefor to assist studies of liver cell function and achieve the result that the liver cells remain viable for an extended period of time. There is no suggestion that the collagen-supported cells could be used as a living tissue, and it is stated in this paper that the cells did not invade the gel matrix. Contraction of the collagen lattice was observed, but the paper reported that the contraction mechanism was not investigated.

*J. Nat. Cancer Inst.*, Vol 53, No. 4, October 1974, (Worst, P. K. M. et al) reports on the culturing of mouse epidermal cells on collagen gels and the re-implantation thereof. Contamination by fibroblasts was excluded, according to the authors.

Fibroblast cells actually used in the experiments described herein were human foreskin fibroblasts and guinea pig dermal fibroblasts. Fibroblasts from other sources can also be used, and it is believed, in fact, that fibroblasts from any vertebrate animal would be suitable for contracting hydrated collagen lattices.

The incorporation of fibroblast cells into hydrated collagen lattices caused the lattices to contract as trapped water was squeezed out. If the surface on which the lattice was formed was non-wettable, e.g. a hydrophobic plate, the resulting tissue was of regular geometry. On tissue culture plates, some cells migrated from the lattice to the plate surface and contraction of the lattice was not always regular. When a non-wettable surface such as a bacteriological Petri plate was used, the lattice remained nearly a perfect disc as its radius was decreased by the cells.

Fibroblast cells were found homogeneously dispersed throughout collagen lattices and not merely upon the lattices' surface. This simulates, then, the dermal layer of humans and other mammals.

In the absence of cells, lattices underwent no change in radius. For example, conditioned medium prepared by growing $1 \times 10^6$ human foreskin fibroblast cells for five days in nutrient medium caused no contraction when no cells were present.

Contracted collagen lattices with cells resembled the skin or dermis; even when partially contracted, they had reasonable consistency and could be readily handled. When first made up with cells, the lattices were almost transparent but gradually became opaque as water was excluded and the diameter reduced. After a 20—30-fold decrease in lattice area, they had a firm rubbery consistency, a whitish pink tint, and could be stretched somewhat without being torn or deformed.

The initial diameter of a lattice was determined by the plate on which it was formed. Thus, maximal contraction was an arbitrary measure.

Although most contracted hydrated collagen lattices were formed as sheets, other shapes could be formed. Tubes, for example, can be formed by forming the contracted lattice in an annular mould, or a glove of skin can be performed from an appropriate mold.

Human foreskin keratinocytes, obtained in a biopsy, were deposited on the contracted hydrated collagen matrix. The same could have been done, of course, with keratinocytes cultivated *in vitro*. Plating of keratinocytes can be done at the time the matrix gel forms, at any time during the period of contraction of the lattice, or any time after contraction has been completed. Within three days after plating suspensions of dissociated keratinocytes, the cells formed a confluent layer on the lattice surface and the process of keratinization began leading to the formation of a corneal layer which would prevent loss of tissue fluids.

The living tissue described herein is suitable for the treatment of a wound to the skin of a human being or other mammal. It is particularly suitable for massive burns wherein it functions as a skin-equivalent. Contraction of hydrated collagen lattices by fibroblast cells can also be used as an assay for measuring fibroblast function, including contractile power.

In addition to skin-equivalents, wound dressings, etc., the living tissues can be tailored for other applications. For example, collagen lattices contracted by fibroblasts could be employed as carriers for other cell types, such as pancreatic islet cells, in the production of a tissue having pancreas function, particularly one produced from cells obtained from the potential recipient.

Additionally, tubes or other shapes might be prepared from lattices contracted by fibroblasts to form living prostheses.

The invention can be further specifically illustrated by the following examples.

Example 1
Preparation of Collagen Lattices Seeded with Fibroblast Cells

Crude collagen solutions were prepared as follows. Frozen rat tails from 450 gm rats were thawed in 70% EtOH for 20 minutes. The tendon bundles were excised in 70% EtOH in a laminar flow hood. Individual tendons were pulled out of the tendon sheath, minced and placed in dilute acetic acid (1:1,000) using 250 ml per tail. This solution was left standing for 48 hours at 4°C at which point the minced tendons had swelled to occupy the total volume. This viscous solution was centrifuged at 23 k rpm in a Beckman L ultracentrifuge in an SW25 rotor for 1 hour. The supernatant was drawn off and stored at 4°C as crude collagen solution (Protein "C").

Refined collagen solution was prepared by mixing crude collagen solution with 0.1*M* NaOH in a 6:1 ratio to neutralize the acetic acid, upon which collagen precipitated. This solution was centrifuged at 1500 rpm for 5 minutes in a clinical centrifuge. The supernatant was discarded and an equal volume of fresh acetic acid (1:1,000) was introduced to resolubilize the collagen. This solution was stored at 4°C as refined collagen solution (Protein "R").

Protein concentration was determined by the method of Lowry et al. See Lowry, O. H., Roseborough, N. J., Farr, N. J. and Randall, R. J., *J. Biol. Chem.*, 193, 265—275 (1951).

Protein lattices were prepared in 60 mm Falcon bacteriological dishes to which fibroblasts adhere poorly. Each dish contained: 1.0 ml 5X McCoy's 5a medium, 1.0 ml Fetal Calf Serum, 0.25 ml 0.1*M* NaOH, 1.5 ml collagen solution, and 1.0 ml fibroblasts suspended in 1X McCoy's medium. The dishes were first filled with the above volume of McCoy's medium, serum and NaOH and then set aside until the fibroblast suspension was prepared. Speed was important in simultaneous addition of collagen solution and fibroblasts since the gel started setting immediately. Dishes were placed in an incubator at 37°C in a 5% $CO_2$ atmosphere. Gels incorporating the fibroblasts were completely set after 10 minutes.

The fibroblasts employed were human foreskin fibroblasts, strain 1519, obtained from the Human Genetic Cell Repository at the Institute for Medical Research in Camden, New Jersey. These cells were grown and maintained in McCoy's 5a modified medium with 20% serum, penicillin, and streptomycin. The cultures were free of mycoplasma. The M.I.T. Cell Culture Center prepared and froze cells of every tenth population doubling level (PDL).

To measure lattice diameters, the dishes were placed on top of a transparent metric ruler on a dark background. Optimum visibility of gel edges was obtained by shining white light horizontally against the edge of the dish. Contracted gels were well formed discs; they showed very slight differences of diameter at various points. The average of the major and minor axes was taken as the diameter.

Example 2

Measurement of Contraction of Hydrated Collagen Lattice by Fibroblast Cells

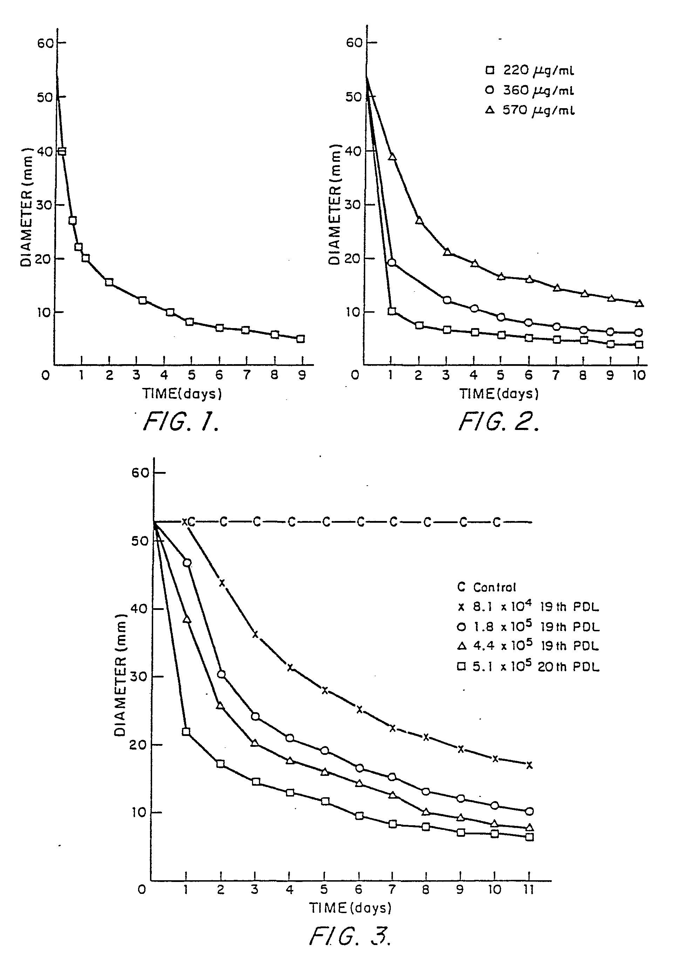

The contraction of a hydrated collagen lattice prepared according to the procedures of Example 1 and containing 570 µg/ml of Protein "C" by 7.5 × 10⁶ human foreskin fibroblasts, strain 1519, 19th PDL, was determined. Medium was changed in the dish on the first, fourth and eighth days. The data obtained are plotted in FIG. 1 which indicates a 112-fold reduction of lattice area in a little over seven days. Within one day, there had been a seven-fold area contraction.

Example 3

Contraction of Hydrated Collagen Lattices of Different Protein Concentration

The effect of protein concentration in hydrated collagen lattices on contraction of the lattice was determined as follows. Three hydrated collagen lattices were prepared according to the procedures of Example 1 except that each contained a different concentration of Protein "R". Human foreskin fibroblasts, strain 1519, 19th PDL were employed and medium was changed on the fourth day.

The data obtained are plotted in FIG. 2 where it can be seen that the rate of lattice contraction varied inversely with gel protein concentration. The area differences became of lesser magnitude as time went on.

Example 4

Effect of Number of Cells on Contraction of Hydrated Collagen Lattices

The effect of the number of cells on contraction of hydrated collagen lattices was determined as follows. A number of hydrated collagen lattices containing 720 µg/ml of Protein "R" were prepared according to the procedure of Example 1. Human foreskin fibroblasts, strain 1519, were employed and the medium in each of the cultures was changed in the third, seventh and tenth days.

Controls were employed to which no cells were added. In addition, four series of experiments were run in which varying numbers of cells were added. The data obtained are plotted in Fig. 3 in which each point represents the average of the contraction of three or four lattices. Deviations are not shown because they were all very small ($< ±1.0$ mm).

As can be seen, the number of cells did have an effect on the rate of gel contraction but this difference in contraction became less significant as a function of time. Gel diameters approached a common small number for concentrations above some minimum value. Below the minimum value, the relationship between rate of gel contraction and cell number was distinctly non-linear. The lattices with 8.1 × 10⁴ cells did not begin to contract for 24 hours. These sparsely populated gels lagged well behind the more densely populated ones throughout the period of the experiments.

Example 5

Contractile Capacity in Hydrated Collagen Lattices of Cells of Different PDL

The contractile capacity in hydrated collagen lattices of cells of different population doubling level (PDL), that is cells which had undergone different numbers of cell divisions, was determined as follows. Cultures were formed of hydrated collagen lattices prepared according to the procedure of Example 1 and containing 720 µg/ml Protein "R". Medium was changed on the third, seventh and tenth days.

Control cultures contained no cells. In addition, a series of experiments with cells of different PDL levels were carried out.

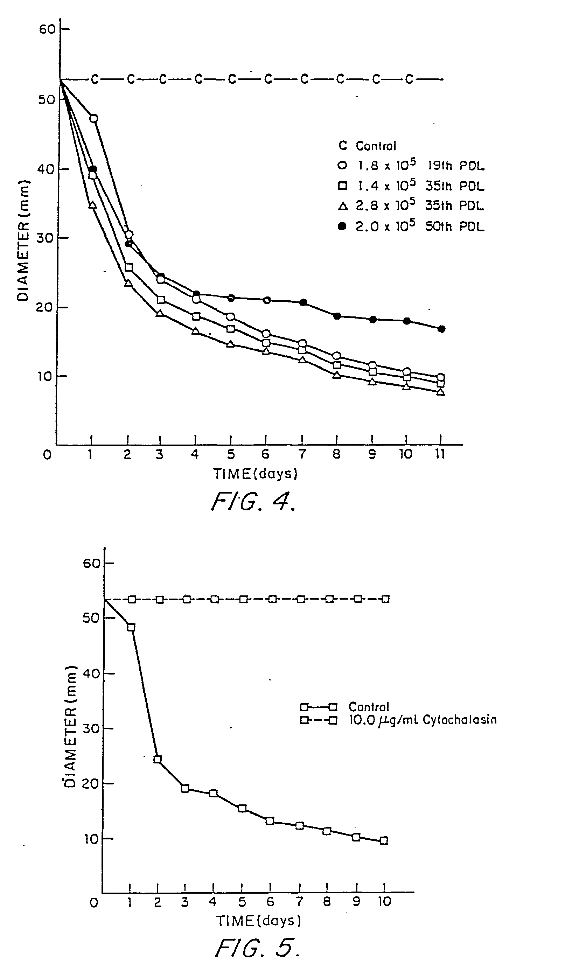

The data collected are plotted in FIG. 4 in which each point represents the average of three or four lattice contractions. Deviations were $< ±1.0$ mm. As can be seen, cells of the 35th PDL performed as well as those of the 19th PDL, but cells of the 50th PDL were unable to contract lattices at a commensurate rate.

Example 6

Effect of Cytochalasin B on Capacity of Cells to Contract a Hydrated Collagen Lattice

The effect of the inhibitor cytochalasin B on the capacity of cells to contract a hydrated collagen lattice was determined as follows. Hydrated collagen lattices were prepared according to Example 1 which contained a Protein "C" content of 570 µg/ml. Fibroblast (human foreskin, strain 1519, 19th PDL) cell concentration in the cultures was $5.0 \times 10^5$. 10.0 µg/ml cytochalasin B was added to each culture and medium was changed on the fourth and eighth days.

Data obtained are plotted in FIG. 5, and as can be seen, this concentration of cytochalasin B completely blocked lattice contraction even at the relatively high cell concentration employed.

### Example 7
Effect of Colcemid on Collagen Lattice Concentration

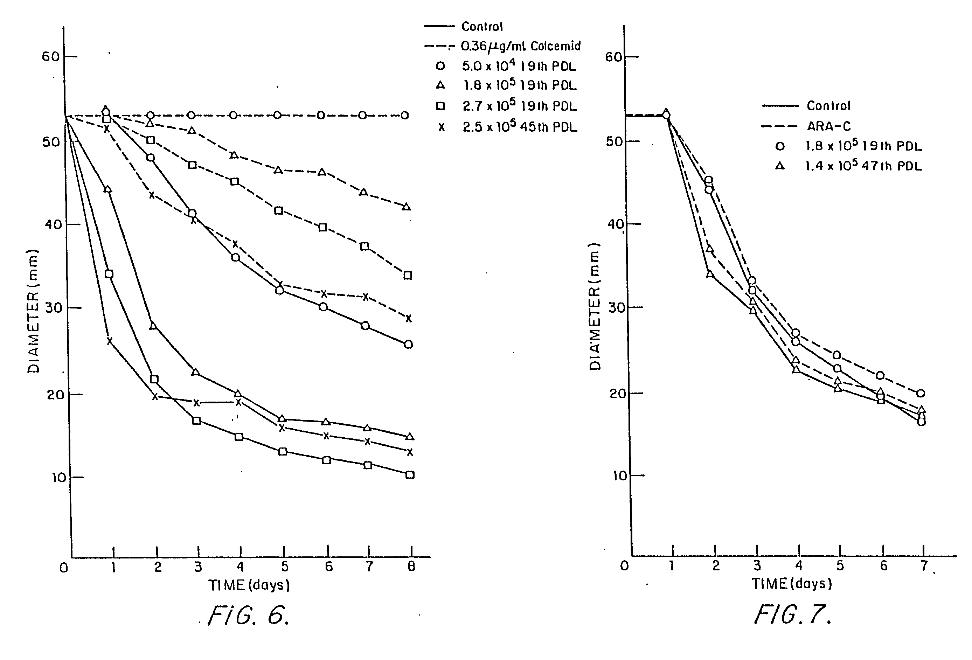

The effect of the inhibitor colcemid on protein lattice contraction was determined as follows. Cultures containing hydrated collagen lattices prepared according to the procedures of Example 1 which contained 570 µg/ml of Protein "C". 0.36 µg/ml colcemid was added to each of these except for the controls which contained no colcemid. The same number of cells was added to both test cultures and control cultures, and data obtained are plotted in FIG. 6. As can be seen, the 45th PDL cells outstripped the performance of 19th PDL cells, while 45th PDL untreated cells lagged behind the 19th PDL untreated cells. It is clear that colcemid can be used to regulate the rate and extent of contraction of the lattices.

### Example 8
Effect of Cytosine Arabinoside on Collagen Lattice Contraction by Cells of Different PDL

The effect of 1.0 µg/ml of cytosine arabinoside on protein lattice contraction by cells of different PDL level was tested as follows. Cultures containing hydrated collagen lattices containing Protein "C" in a concentration 570 µg/ml were prepared. Human foreskin fibroblasts, strain 1519, of the 19th PDL or 47th PDL were added to both controls containing no cytosine arabinoside and test cultures containing cytosine arabinoside. Data obtained are plotted in FIG. 7, where it can be seen that the 47th PDL cells outperformed the lower PDL cells even though they were fewer in number. In these experiments cytosine arabinoside was used to block DNA synthesis and thereby keep the number of cells in the lattice constant.

### Example 9
Formation of Skin-Equivalent Employing Human Foreskin Fibroblasts and Keratinocytes

A hydrated collagen lattice was prepared according to the procedures of Example 1 which contained 500 µg/ml of Protein "C". Human foreskin fibroblasts obtained in a biopsy were removed from a culture plate with a solution of EDTA and trypsin. The suspension of single cells was centrifuged to pellet the cells, after which the cells were resuspended in culture medium and then deposited on top of the hydrated collagen matrix seven days after the fibroblast cells had been introduced. Within three days, the keratinocyte cells had attached to the lattice substrate and the process of keratinization began leading to the formation of an impervious cornium. Histological observations were made by means of electron microscopy.

### Example 10
In Vivo Studies with Skin-Equivalent Employing Guinea Pig Skin Fibroblasts and Keratinocytes

Skin biopsies were taken from guinea pigs and dermis was separated from epidermis surgically. Dermis was dissociated enzymatically into constituent cells which were plated onto tissue culture dishes and allowed to undergo proliferation. Cells from each experimental animal were grown in separate dishes so that their identity was preserved. Tissues were made up *in vitro* by forming contracted-hydrated collagen lattices according to the procedures of Example 1 except employing fibroblasts from the guinea pigs. Some of the lattices, after contraction, were plated according to Example 9 with epidermal cells or keratinocytes taken from second biopsies so that keratinocytes as well as fibroblasts in each graft were from the animal which was to become the recipient of the graft.

Grafts of these skin-equivalents were made to the dorsum of the experimental animals (guinea pigs), and it was found that such grafts were thoroughly integrated at all levels within one week. From below, they had become vascularized; at the level of the dermis, collagen fibrils of the graft were interwoven with those of the surrounding host tissue. In histological sections, the grafts could be distinguished by their fibroblast cell density and by the reduced degree of birefringence as compared with that of surrounding skin when viewed through a polarization microscope. Even those grafts not provided with epidermis were completely covered with an epidermal cell layer (keratinocytes) many cells deep. The layer was continuous with that of the adjacent host skin. It was clear also that dermal wound contraction was inhibited by the presence of the skin-equivalent graft just as it is when an autograft is made.

### Claims

1. A method of producing living tissue characterised by:
   a. forming a hydrated collagen lattice;
   b. bringing fibroblast cells into contact with said collagen lattice; and
   c. maintaining said lattice and said cells under conditions sufficient for said cells to attach to said collagen lattice and to contract said collagen lattice thereby forming living tissue.

2. A method as claimed in claim 1, characterised in that said hydrated collagen lattice is formed on a non-wettable surface.

3. A method of preparing living tissue, characterised by:
   a. forming an acidic solution of collagen;
   b. combining fibroblast cells and nutrient medium with said acidic solution of collagen;
   c. raising the pH of said solution of collagen to a level sufficient to precipitate collagen fibrils into a hydrated collagen lattice; and

d. maintaining said lattice and said cells under conditions sufficient for said cells to attach to said collagen lattice and to contract said collagen lattice thereby forming living tissue.

4. A method as claimed in claim 3, characterised in that steps b and c are done simultaneously.

5. A method of forming skin-equivalent, comprising producing living tissue by the method claimed in claim 1, characterised by plating keratinocyte cells upon said living tissue; and maintaining said living tissue under conditions sufficient for growth of said fibroblast cells and said keratinocyte cells to thereby produce a skin-equivalent.

6. A skin-equivalent characterised by a contracted hydrated collagen lattice containing fibroblast cells therein, and keratinocyte cells supported upon said contracted hydrogen collagen lattice.

## Patentansprüche

1. Verfahren zur Bildung von lebendem Gewebe, gekennzeichnet durch folgende Verfahrensschritte:

a) Herstellung eines hydrierten Kollagengitters;

b) Inberührungbringen von Fibroblasten mit dem Kollagengitter und

c) Aufrechterhalten von solchen Bedingungen für das Kollagengitter und die Fibroblasten, welche dazu ausreichen, daß die Zellen sich an dem Kollagengitter anlagern und dieses kontrahieren, wobei sich lebendes Gewebe bildet.

2. Verfahren nach Anspruch 1, dadurch gekennzeichnet, daß das hydratisierte Kollagengitter auf einer nicht benetzbaren Oberfläche gebildet wird.

3. Verfahren zur Bildung von lebendem Gewebe, gekennzeichnet durch folgende Verfahrensschritte:

a) Bildung einer sauren Lösung von Kollagen;

b) Vereinigen von Fibroblasten und einem Nährmedium mit der sauren Kollagenlösung;

c) Anheben des pH-Wertes der Kollagenlösung auf solches Niveau, daß Kollagenfibrilien zu einem Hydratisierten Kollagengitter ausfallen und

d) Aufrechterhalten von solchen Bedingungen für das Kollagengitter und die Fibroblasten, welche dazu ausreichen, daß sich die Fibroblasten an dem Kollagengitter anlagern und dieses kontrahieren, wobei lebendes Gewebe gebildet wird.

4. Verfahren nach Anspruch 3, dadurch gekennzeichnet, daß die Verfahrensschritte b) und c) gleichzeitig ausgeführt werden.

5. Verfahren zur Herstellung eines Hautersatzes, wobei lebendes Gewebe nach dem Verfahren gemäß Anspruch 1 gebildet wird, gekennzeichnet durch Beschichtung des lebenden Gewebes mit Keratozyten und Aufrechterhalten von solchen Bedingungen für das lebende Gewebe, welche dazu ausreichen, daß die Fibroblasten und die Keratozyten wachsen, so daß sich ein Hautersatz bildet.

6. Hautersatz, gekennzeichnet durch ein kontrahiertes hydratisiertes Kollagengitter, welches Fibroblasten enthält, sowie durch Keratozyten, welche auf dem kontrahierten hydratisierten Kollagengitter angeordnet sind.

## Revendications

1. Procédé pour produire un tissu vivant, caractérisé en ce qu'il consiste:

a) à former un réseau de collagène hydraté,

b) à mettre en contact des fibroblastes avec ledit réseau de collagène,

c) à maintenir ledit réseau et lesdites cellules dans des conditions suffisantes pour que lesdites cellules se fixent audit réseau de collagène et contractent ledit réseau de collagène en formant ainsi un tissu vivant.

2. Procédé selon la revendication 1, caractérisé en ce que ledit réseau de collagène hydraté est formé sur une surface non mouillable.

3. Procédé pour préparer un tissu vivant caractérisé en ce qu'il consiste:

a) à former une solution acide de collagène,

b) à combiner des fibroblastes et un milieu nutritif avec ladite solution acide de collagène,

c) à élever le pH de ladite solution de collagène à une valeur suffisante pour précipiter des fibrilles de collagène dans un réseau de collagène hydraté,

d) à maintenir ledit réseau et lesdites cellules dans des conditions suffisantes pour que lesdites cellules se fixent audit réseau de collagène et contractent ledit réseau de collagène en formant ainsi un tissu vivant.

4. Procédé selon la revendication 3, caractérisé en ce que les étapes b et c sont effectuées simultanément.

5. Procédé pour fabriquer un substitut ou succédané de peau comprenant la production de tissu vivant par le procédé selon la revendication 1, caractérisé en ce que l'on applique des kératinocytes sur ledit tissu vivant; et on maintient ledit vivant dans des conditions suffisantes pour la croissance desdits fibroblastes et desdits kératinocytes pour produire ainsi un substitut de peau.

6. Substitut de peau, caractérisé par un réseau de collagène hydraté contracté contenant des fibroblastes, et des kératinocytes supportés sur ledit réseau de collagène hydraté contracté.

FIG. 1.

FIG. 2.

FIG. 3.

FIG. 4.

FIG. 5.

FIG. 7.

FIG. 6.

3